# FASCICULE DE BREVET EUROPEEN

(11) **EP 1 931 796 B1**
(45) Date de publication et mention de la délivrance du brevet: **09.02.2011**
(21) Numéro de dépôt: 06793015.6
(22) Date de dépôt: 25.08.2006
(51) Int. Cl.: C12Q 1/04

(54) **DÉTECTION DES SALMONELLES LACTOSE+**
NACHWEIS VON SALMONELLA-LACTOSE+
DETECTION OF SALMONELLA LACTOSE+

(30) Priorité: 26.08.2005 FR 0508770
(43) Date de publication de la demande: 18.06.2008
(73) Titulaire: Rambach, Alain, F-75006 Paris (FR)
(72) Inventeur: Rambach, Alain, F-75006 Paris (FR)
(74) Mandataire: Warcoin, Jacques
(86) Numéro de dépôt international: PCT/EP2006/065684
(87) Numéro de publication internationale: WO 2007/023185

(56) Documents cités:
- EP-A- 0 326 635
- WO-A-92/17607
- WO-A-99/41409
- WO-A-99/50438

## Description

La présente invention se rapporte à un milieu de détection et/ou d'identification de Salmonelles.

La détection et l'identification de la bactérie *Salmonella,* pathogène pour l'homme, est un problème majeur de la bactériologie médicale et de la surveillance de l'hygiène agro-alimentaire.

Cette bactérie est notamment responsable chez l'homme de la fièvre typhoïde et d'intoxications alimentaires.

Dans le cas d'épidémies transmises par des élevages de poules, les volailles infestées au niveau du tractus intestinal ne sont pas malades mais constituent un réservoir de Salmonelles. Celles-ci peuvent être ensuite propagées, notamment par les oeufs, dans l'alimentation. D'ailleurs, *Salmonella* est une bactérie à déclaration obligatoire.

Il est donc nécessaire de prévoir la mise en oeuvre à grande échelle de la détection des sites infectés et plus particulièrement des fermes infectées par les Salmonelles pour réduire ces épidémies et limiter les contaminations.

La détection des Salmonelles est habituellement réalisée sur un milieu gélosé d'isolement sélectif pour entérobactéries permettant la différenciation des entérobactéries pathogènes et la détection des colonies suspectes de Salmonelles. Un milieu de détection et/ou d'identification idéal doit permettre la croissance d'entérobactéries, la différenciation des diverses espèces présentes afin de permettre l'identification ultérieure d'une colonie de chaque type, et la détection des colonies suspectes de Salmonelles.

Plus particulièrement, il est important de détecter les Salmonelles, qui pour la plupart d'entre elles sont lactose⁻, il est important de pouvoir également détecter les souches de Salmonelles lactose⁺ qui, tout en représentant un faible pourcentage des souches totales de Salmonelles rencontrées, notamment en agro-alimentaire, sont très souvent identifiées, de façon erronée, en tant que *Escherichia coli* et donnent donc lieu à des faux positifs.

En effet, il a été longuement considéré que la fermentation en présence de lactose permettait de différencier les bactéries *Salmonella* des autres *Enterobacteriaceae.* Il était en effet connu depuis fort longtemps que *Escherichia coli* présentait le caractère lactose⁺ tandis que les Salmonelles présentaient le caractère lactose⁻. La découverte de souches de Salmonelles lactose⁺ a donc mis en évidence le manque de fiabilité des tests basés sur le caractère lactose des entérobactéries et a mis en exergue l'importance de la détection des Salmonelles lactose⁺.

L'objet de la présente invention est de permettre de réaliser des tests de routine pour la détection et l'identification des Salmonelles par exemple des méthodes de surveillance de la qualité microbiologique de l'eau par des méthodes de trempage, ou la surveillance de la qualité microbiologique des usines agro-alimentaires par des méthodes de contact, le diagnostique ou encore la recherche de microorganismes pathogènes par les méthode microbiologiques traditionnelles sur boîte de Petri, sur milieu solide ou sur milieu sélectif semi-solide, à l'aide de milieux conformes à l'invention.

L'objet de la présente invention est d'augmenter la sensibilité et la sélectivité de la détection et la différenciation des Salmonelles dans un échantillon contaminé au moyen d'un milieu de culture qui, tout en permettant la mise en évidence de l'activité estérase propre à ces bactéries, permet une différenciation accrue des Salmonelles parmi les autres entérobactéries, y compris les Salmonelles lactose⁺.

L'homme du métier connaît déjà les milieux de culture destinés à mettre en évidence des Salmonelles au moyen d'agents chromogènes (brevet FR 2 197 028, Rambach) mais si ce type de milieu permet de différencier les Salmonelles des autres entérobactéries, il ne distingue pas nécessairement les Salmonelles lactose⁺.

Les milieux de l'art antérieur sont d'ailleurs généralement fondés sur le caractère lactose⁻ ou béta-galactosidase⁻ des Salmonelles.

Le milieu de détection et/ou d'identification des Salmonelles de la présente invention comprend un chromogène lié au caprylate ou l'un de ses dérivés, substrat des estérases, qui sera libéré dans le milieu sous l'action de ces enzymes permettant de ce fait, l'identification des colonies de Salmonelles.

Cependant, afin d'augmenter la sélectivité et la sensibilité du milieu de l'invention permettant alors la détection des Salmonelles lactose⁺, ledit milieu comprend également un détergent de la famille des nonylphénol éthoxylates

Il a été trouvé d'une manière inattendue, que l'addition d'un tel détergent au milieu de l'invention, à une concentration appropriée et compatible avec une bonne croissance des Salmonelles, permettait non seulement la détection plus aisée des colonies de Salmonelles mais également la détection des Salmonelles lactose⁺.

Parmi les nonylphénol éthoxylates utilisables dans le cadre de la présente invention, on peut notamment citer les Tergitol® NP (commercialisés par The Dow Chemical Company) et en particulier le Tergitol® NP-7 et le Tergitol® NP-9 également appelé Igepal® CO-630 ou Triton® N-60. Il s'agit du α-(nonylphenyl)-ω-hydroxypoly(oxy-1,2-éthanediyl).

D'une manière préférentielle, le détergent approprié sera utilisé à une concentration comprise entre 0,5 et 2,5 g/l, de préférence entre 1 et 2 g/l et plus préférentiellement encore entre 1,3 et 1,7 g/l.

Selon un mode de réalisation préféré de l'invention, le composé chromogène est un ester de l'acide caprylique, avantageusement choisi dans le groupe comprenant les dérivés indolyl caprylate et les dérivés hydroxy-quinoline caprylate ainsi que leurs sels. Parmi ces dérivés, on peut citer plus particulièrement les dérivés halogéno-indolyl caprylate (bromo-indolyl caprylate, chloro-indolyl caprylate, fluoro-indolyl caprylate, iodo-indolyl caprylate, dichloro-indolyl caprylate, chloro-bromo-indolyl caprylate, tri-chloro-indolyl-caprylate), les dérivés méthyl-indoxyl caprylate.

Plus particulièrement encore, l'ester de l'acide caprylique est choisi dans le groupe comprenant les dérivés 6-chloro-indolyl caprylate, 5-bromo-indolyl caprylate, 3-bromo-indolyl caprylate, 6-fluoro-indolyl caprylate, 5-iodo-indolyl caprylate, 4,6-dichloro-indolyl caprylate, 6,7-dichloro-indolyl caprylate, 5-bromo-4-chloro-indolyl caprylate, 5-bromo-6-chloro-indolyl caprylate, 4,6,7-trichloro-indolyl caprylate, N-méthyl-indolyl caprylate et 8-hydroxy-quinoline caprylate.

Le chromogène est utilisé, de manière préférentielle, à une concentration comprise entre 0,1 à 0,4 g/l, de préférence entre 0,15 à 0,3 g/l.

L'homme du métier est tout à fait en mesure de compléter le milieu de l'invention dans le but d'éliminer les bactéries autres que les Salmonelles, par exemple par la mise en évidence, chez ces autres bactéries, de caractères absents chez les Salmonelles qui sont notamment béta-glucosidase. Par conséquent, le milieu de l'invention pourra également comprendre des dérivés de béta-glucoside liés à un chromophore différent de celui utilisé pour la mise en évidence des Salmonelles.

Le milieu de la présente invention permet donc de détecter et d'identifier une colonie colorée de *Salmonella,* de façon accrue, même en présence de milliers d'autres colonies, sur un milieu solide ou semi-solide. En fait, les colonies de Salmonelles détectées par le milieu de l'invention sont colorées d'une certaine couleur tandis que les autres microorganismes apparaissent de couleurs différentes ou incolores.

La détection des colonies de Salmonelles dans le cadre de la présente invention est directe en ce sens qu'elle ne nécessite aucun acte ou intervention ultérieur(e) à la fermentation des colonies telle que l'utilisation d'un révélateur quelconque ou celui d'une lumière particulière.

La présente invention concerne également un procédé de détection et/ou d'identification de Salmonelles dans un prélèvement ou un échantillon quelconque comprenant l'inoculation du milieu de culture de l'invention avec l'échantillon ou le prélèvement à tester et en la mise en évidence de la coloration caractéristique de la présence de colonies de Salmonelles.

En fonction de la forme et la présentation du milieu et en fonction du type de protection effectuée, l'inoculation sera réalisée de préférence par contact, par trempage, en surface ou dans la masse.

L'exemple ci-après est destiné à illustrer la présente invention et ne peut aucunement être considéré comme limitatif.

### Exemple : détection de Salmonelles

I. Selon une première série d'expérimentations : plusieurs milieux ont été testés pour la détection et la différenciation de *E.coli* (composant principal de la flore commensale des échantillons dans lesquels on recherche la présence de Salmonelles) et des *Salmonella* (en particulier des *Salmonella* lactose⁺).
Chacun desdits milieux est constitué de la même base à laquelle sont ajoutés d'autres composés comme indiqué ci-dessous.
Base (g/l) :
Extrait de viande ou levure 3
Peptone 5
Chlorure de sodium 5
Agar 15
5-bromo-6-chloro-3-indolyl caprylate 0,2
5-bromo-4-chloro-3-indolyl beta glucoside 0,1
Milieu A (g/l) :
Base
+ Sodium deoxycholate 2,5
Milieu B (g/l) :
Base
+ Sodium deoxycholate 1,5
+ Triton XL-80N deoxycholate 1,5
Milieu C de l'invention (g/l) :
Base
+ Sodium deoxychotate 1,5
+ Tergitol NP-9 deoxycholate 1,5

| **Milieu** | **A** | **B** | **C** |
|---|---|---|---|
| *Salmonella* spp | + | + | ++ |
| *Salmonella* lactose positive | + | + | ++ |
| *E. coli* | + | + | incolore |

| | | | |
|---|---|---|---|
| « + » représente l'intensité de la coloration mauve obtenue. | | | |

Seul le milieu de l'invention (C) permet de distinguer les *Salmonella* spp et les *Salmonella* lactose⁺ des *E. coli.*
II. Une autre expérimentation a consisté à comparer le susdit milieu C de l'invention avec le milieu XLT4 (Xylose-Lysine-Tergitol (Niaproof)4) pour la détection des *Salmonella,* y compris des souches lactose⁺.

| **Milieu** | **XLT4** | **C** |
|---|---|---|
| *Salmonella* spp (ATCC 35640) | + | + |
| *Salmonella* senftenberg lactose⁺ (321) | - | + |

| | | |
|---|---|---|
| Seul le milieu de l'invention (C) permet de détecter toutes les souches de *Salmonella.* | | |

## Revendications

1. Milieu de détection et/ou d'identification de Salmonelles comprenant un substrat chromogène de la caprylate estérase ou l'un de ses sels et un détergent de la famille des nonylphénols éthoxylates.

2. Milieu selon la revendication 1, **caractérisé en ce que** le substrat chromogène de la caprylate estérase est un ester de l'acide caprylique.

3. Milieu selon la revendication 2, **caractérisé en ce que** l'ester de l'acide caprylique est choisi parmi les dérivés indolyl-caprylate et les dérivés hydroxy-quinoline-caprylate.

4. Milieu selon la revendication 3, **caractérisé en ce que** l'ester de l'acide caprylique est choisi dans le groupe comprenant les dérivés halogéno-indolyl caprylate (bromo-indolyl caprylate, chloro-indolyl caprylate, fluoro-indolyl caprylate, iodo-indolyl caprylate, dichloro-indolyl caprylate, chloro-bromo-indolyl caprylate, tri-chloro-indolyl caprylate), les dérivés méthyl-indoxyl caprylate.

5. Milieu selon la revendication 3 ou 4, **caractérisé en ce que** l'ester de l'acide caprylique est choisi dans le groupe comprenant les dérivés 6-chloro-indolyl caprylate, 5-bromo-indolyl caprylate, 3-bromo-indolyl caprylate, 6-fluoro-indolyl caprylate, 5-iodo-indolyl caprylate, 4,6-dichloro-indolyl caprylate, 6,7-dichloro-indolyl caprylate, 5-bromo-4-chloro-indolyl caprylate, 5-bromo-6-chloro-indolyl caprylate, 4,6,7-trichloro-indolyl caprylate, N-méthyl-indolyl caprylate et 8-hydroxy-quinoline caprylate.

6. Milieu selon la revendication 5, **caractérisé en ce que** le dérivé indolyl caprylate est le 5-bromo-6-chloro-3-indolyl caprylate.

7. Milieu selon l'une des revendications 1 à 6, **caractérisé en ce que** le détergent l'α-(nonyphenyl)-ω-hydroxypoly(oxy-1,2-éthanediyl)

8. Milieu selon la revendication 7, **caractérisé en ce que** l'(x-(nonyphenyl)-ω)-hydroxypoly(oxy-1,2-éthanediyl) est présent dans ledit milieu à une concentration comprise entre 0,5 et 2,5 g/l, de préférence entre 1 et 2 g/l et plus préférentiellement encore entre 1,3 et 1,7 g/l.

9. Procédé de détection et/ou d'identification de Salmonelles dans un échantillon comprenant les étapes suivantes :
- inoculation d'un milieu selon l'une des revendications 1 à 8 avec ledit échantillon, et
- mise en évidence de la coloration caractéristique de la présence des Salmonelles.

## Claims

1. Medium for the detection and/or identification of Salmonella comprising a chromogenic substrate of caprylate esterase or one of its salts and a detergent from the nonylphenol ethoxylates family.

2. Medium according to claim 1, **characterised in that** the chromogenic substrate of caprylate esterase is an ester of caprylic acid.

3. Medium according to claim 2, **characterised in that** the caprylic acid ester is chosen among indolyl caprylate derivatives and hydroxy-quinoline caprylate derivatives.

4. Medium according to claim 3, **characterised in that** the caprylic acid ester is chosen from the group comprising halogeno-indolyl caprylate derivatives (bromo-indolyl caprylate, chloro-indolyl caprylate, fluoro-indolyl caprylate, iodo-indolyl caprylate, dichloro-indolyl caprylate, chloro-bromo-indolyl caprylate, tri-chloro-indolyl caprylate) and methyl-indoxyl-caprylate derivatives.

5. Medium according to claim 3 or 4, **characterised in that** the caprylic acid ester is chosen from the group comprising the derivatives of 6-chloro-indolyl-caprylate, 5-bromo-indolyl-caprylate, 3-bromo-indolyl-caprylate, 6-fluoro-indolyl-caprylate, 5-iodo-indolyl-caprylate, 4,6-dichloro-indolyl-caprylate, 6,7-dichloro-indolyl-caprylate, 5-bromo-4-chloro-indolyl-caprylate, 5-bromo-6-chloro-indolyl-caprylate, 4,6,7-trichloro-indolyl-caprylate, N-methyl-indolyl-caprylate and 8-hydroxy-quinoline caprylate.

6. Medium according to claim 5, **characterised in that** the indolyl caprylate derivative is 5-bromo-6-chloro-3-indolyl caprylate.

7. Medium according to one of claims 1 to 6, **characterised in that** the detergent is α-(nonylphenyl)-ω-hydroxypoly(oxy-1,2-ethanediyl).

8. Medium according to claim 7, **characterised in that** α-(nonylphenyl)-ω-hydroxypoly(oxy-1,2-ethanediyl) is present in said medium at a concentration comprised between 0.5 g/l and 2.5 g/l, preferably between 1 g/l and 2 g/l and still more preferentially between 1.3 g/l and 1.7 g/l.

9. Method for the detection and/or identification of Salmonella in a sample, comprising the following steps:
- inoculation of a medium according to one of claims 1 to 8 with said sample, and
- detection of the colour characteristic of the presence of Salmonellas.

## Patentansprüche

1. Medium zum Nachweis und/oder zur Identifikation von Salmonellen, umfassend ein chromogenes Substrat der Caprylatesterase oder eines seiner Salze und ein Detergens der Familie der Nonylphenolethoxylate.

2. Medium nach Anspruch 1, **dadurch gekennzeichnet, dass** das chromogene Substrat der Caprylatesterase ein Caprylsäureester ist.

3. Medium nach Anspruch 2, **dadurch gekennzeichnet, dass** der Caprylsäureester ausgewählt ist aus Indolylcaprylat-Derivaten und Hydroxy-chinolincaprylat-Derivaten.

4. Medium nach Anspruch 3, **dadurch gekennzeichnet, dass** der Caprylsäureester ausgewählt ist aus der Gruppe umfassend Halogenindolylcaprylat-Derivate (Bromindolylcaprylat, Chlorindolylcaprylat, Fluorindolylcaprylat, lodindolylcaprylat, Dichlorindolylcaprylat, Chlorbromindolylcaprylat, Trichlorindolylcaprylat), Methylindoxylcaprylat-Derivate.

5. Medium nach Anspruch 3 oder 4, **dadurch gekennzeichnet, dass** der Caprylsäureester ausgewählt ist aus der Gruppe umfassend die Derivate 6-Chlorindolylcaprylat, 5-Bromindolylcaprylat, 3-Bromindolylcaprylat, 6-Fluorindolylcaprylat, 5-Iodindolylcaprylat, 4,6-Dichlorindolylcaprylat, 6,7-Dichlorindolylcaprylat, 5-Brom-4-chlorindolylcaprylat, 5-Brom-6-chlorindolylcaprylat, 4,6,7-Trichlorindolylcaprylat, N-Methylindolylcaprylat und 8-Hydroxy-chinolincaprylat.

6. Medium nach Anspruch 5, **dadurch gekennzeichnet, dass** das Indolylcaprylat-Derivat 5-Brom-6-chlor-3-indolylcaprylat ist.

7. Medium nach einem der Ansprüche 1 bis 6, **dadurch gekennzeichnet, dass** das Detergens a-(Nonylphenyl)-w-hydroxypoly(oxy-1,2-ethandiyl) ist.

8. Medium nach Anspruch 7, **dadurch gekennzeichnet, dass** das α-(Nonylphenyl-ω-hydroxypoly(oxy-1,2-ethandiyl) in dem Medium in einer Konzentration zwischen 0,5 und 2,5 g/l, vorzugsweise zwischen 1 und 2 g/l und bevorzugter noch zwischen 1,3 und 1,7 g/l einschließlich vorliegt.

9. Verfahren zum Nachweis und/oder zur Identifikation von Salmonellen in einer Probe, umfassend die folgenden Schritte:
- Inokulation eines Mediums nach einem der Ansprüche 1 bis 8 mit der Probe und
- Nachweisen der charakteristischen Färbung der Anwesenheit von Salmonellen.
